Europäisches Patentamt

**European Patent Office**

Office européen des brevets

(11) Numéro de publication: **0 174 216 B1**

# FASCICULE DE BREVET EUROPEEN

(45) Date de publication de fascicule du brevet:
**07.08.91**

(51) Int. Cl.⁵: **A61F 5/01, A61L 15/07**

(21) Numéro de dépôt: **85401068.3**

(22) Date de dépôt: **30.05.85**

(54) **Nouveaux éléments pour la réalisation d'orthèses et leurs procédés d'assemblage.**

(30) Priorité: **02.08.84 FR 8412257**

(43) Date de publication de la demande:
**12.03.86 Bulletin 86/11**

(45) Mention de la délivrance du brevet:
**07.08.91 Bulletin 91/32**

(84) Etats contractants désignés:
**AT BE CH DE FR GB IT LI LU NL SE**

(56) Documents cités:
| | |
|---|---|
| DE-A- 1 154 595 | DE-A- 2 018 334 |
| DE-C- 37 437 | FR-A- 480 769 |
| FR-A- 2 174 189 | FR-A- 2 208 640 |
| FR-A- 2 284 634 | FR-A- 2 350 091 |
| FR-A- 2 493 141 | GB-A- 1 499 807 |
| GB-A- 1 564 904 | US-A- 2 559 473 |
| US-A- 3 827 431 | US-A- 3 929 140 |
| US-A- 4 312 335 | |

(73) Titulaire: **PROTEOR, Société Anonyme dite:**
**11, rue des Buttes**
**F-21100 Dijon(FR)**

(72) Inventeur: **Berthet, Marcel**
**23 A, rue de la Libération**
**F-21240 Talant(FR)**
Inventeur: **Pierron, Gilbert**
**18, rue du Fg St Georges**
**F-21250 Seurre(FR)**
Inventeur: **Palfray, Michel**
**Route de Pagny**
**F-21250 Seurre(FR)**

(74) Mandataire: **Jolly, Jean-Pierre et al**
**Cabinet Jolly 54, rue de Clichy**
**F-75009 Paris(FR)**

## Description

La présente invention concerne de nouveaux éléments pour la réalisation d'orthèses.

On sait que les orthèses sont utilisées pour maintenir, assister, consolider un membre ou un segment de membre d'une personne, en vue de lui redonner une fonction.

De nombreux types d'orthèses ont été proposés dans la technique. Elles comprennent généralement une armature constituée d'éléments longitudinaux, dits encore "attelles" latérales ou "tuteurs", disposés sur la face externe et éventuellement sur la face interne du membre appareillé. Ces éléments sont réunis, le cas échéant, par des bandes transversales ou "embrasses", qui épousent la forme du membre en lui procurant, si nécessaire, un appui, et qui assurent l'écartement désiré pour les attelles, tout en conférant à l'orthèse une rigidité suffisante. Les montants supérieurs et inférieurs peuvent être réunis par un système d'articulation, éventuellement muni d'un moyen de verrouillage.

L'armature des orthèses est habituellement métallique et est garnie de cuir, de tissu ou de matière plastique.

La réalisation d'une orthèse de ce type est complexe et coûteuse, car elle exige un travail du métal pour sa mise en forme, un assemblage par soudure et/ou par rivetage des différents éléments et diverses opérations de finition des parties assemblées.

En outre, bien qu'habituellement réalisées en acier ou en un alliage léger, elles sont relativement lourdes pour les patients qui sont amenés à les utiliser.

Enfin, du fait de la complexité de leur élaboration, leur réalisation implique des délais de livraison relativement longs aux personnes hospitalisées, ce qui retarde leur sortie des hôpitaux ou cliniques et accroît notablement les frais d'hospitalisation.

On a déjà proposé, dans la technique antérieure, d'utiliser certaines résines thermoplastiques en remplacement du plâtre pour immobiliser des membres fracturés (voir FR-A-2 208 640 et 2 284 634). Les matières thermoplastiques utilisées se ramollissent toutefois à des températures beaucoup trop basses pour qu'elles puissent être utilisées pour la réalisation d'orthèses. En outre, bien qu'il ait été proposé de les renforcer à l'aide de courtes fibres de verre et de charges de silice, leur résistance mécanique et leur élasticité sont insuffisantes pour la fabrication d'éléments d'orthèses, tels que des attelles ou des embrasses.

La présente invention vise à remédier à ces inconvénients de la technique connue en proposant de nouveaux constituants d'orthèses, qui simplifient de façon appréciable la fabrication des orthèses, qui réduisent par conséquent leur délai de livraison et leur coût et qui permettent de réaliser des orthèses beaucoup plus légères et résistantes que celles de la technique antérieure.

A cet effet, l'invention a pour objet des attelles et des embrasses pour la constitution d'orthèses, caractérisées en ce qu'elles sont réalisées en une résine thermoplastique, choisie dans le groupe constitué par les polyesters et les résines acryliques, dans laquelle sont noyées des fibres tissées de carbone, de bore ou d'aramide.

Les fibres rentrant dans la composition de ces éléments d'orthèses seront tissées, afin d'éviter un délaminage éventuel. Avantageusement, ces fibres seront tissées sous forme de tresses. L'orientation des fibres sera choisie de façon à obtenir une grande résistance des éléments et un thermoformage aisé.

Les attelles et les embrasses pourront provenir d'éléments standards plats, que l'on amène à épouser le contour du membre à appareiller par une simple mise en forme à chaud. Ils peuvent également être découpés pour être amenés à la dimension désirée.

Les éléments d'orthèses conformes à l'invention peuvent aussi posséder d'emblée la forme appropriée au membre à appareiller. Pour les réaliser, on utilisera dans ce cas un moule déformable, auquel on conférera un profil correspondant à celui du membre à équiper, on y introduira la matière composite avant polymérisation de la résine entrant dans sa composition et l'on procèdera ensuite à la polymérisation de cette résine.

Les attelles ainsi réalisées peuvent être rendues solidaires, par l'une et/ou l'autre de leurs extrémités, de raccords faisant partie d'articulations d'un type usuel.

Les "attelles" ou "montants" ainsi réalisés pourront comporter au moins une partie en saillie formant voile ou nervure sur tout ou partie de leur longueur, contre laquelle pourront être amenés à la longueur désirée et assemblées une ou des embrasses, les parties inutilisées de ladite nervure pouvant ensuite être éliminées par découpage.

L'attelle pourra aussi avoir elle-même, sur tout ou partie de sa longueur une section en U, dans laquelle sera engagée, sur la longueur désirée, l'extrémité de l'embrasse à ajuster.

L'attelle pourra être constituée de deux ou plusieurs lamelles parallèles réunies en au moins deux points sur leur longueur et entre lesquelles pourra être engagée à la longueur voulue l'extrémité de l'embrasse.

Des attelles conformes à l'invention pourront aussi être formées d'un faisceau de deux ou plusieurs lames accolées, réunies en au moins un point de leur longueur. L'extrémité de l'embrasse, coupée à la longueur voulue, pourra être engagée entre les faisceaux de l'attelle. Un élément du faisceau pourra être découpé sur une partie de sa longueur afin de faciliter le passage de l'embrasse. Cette variante permet la mise en forme des attelles à froid par glissement relatif des éléments du faisceau.

Il est bien entendu qu'une attelle peut comporter une ou plusieurs des variantes décrites ci-dessus.

L'assemblage des "attelles" ou "montants" avec la ou les embrasses de cuissard ou jambière, brassard ou avant-brassard, peut être réalisé par collage, rivetage, boulonnage, polymérisation avec un tissu de carbone imprégné de résine, ou tout autre moyen connu de la technique.

On notera que, dans toutes ces variantes de mise en oeuvre de l'invention, l'embrasse peut être aisément mise à dimension et fixée sur l'attelle dans la position et à la longueur désirées ; les parties superflues de l'embrasse pourront elles-mêmes être découpées après mise à longueur et avant assemblage. Pour certains modes de réalisation, l'embrasse pourra être constituée d'un faisceau de deux ou plusieurs lames accolées réunies en au moins un point de leur longueur, entre lesquelles une partie de l'attelle -une nervure, par exemple- ou le corps de l'attelle proprement dite pourra être engagé de façon à permettre une mise à longueur précise de l'embrasse avant assemblage de celle-ci avec l'attelle.

La mise en forme des "attelles" peut être faite par thermoformage. Différents moyens connus de la technique peuvent être employés à cet effet, notamment le formage sur un moulage positif du membre du patient à appareiller.

Un dispositif de formage des attelles sera également décrit ci-après. Ce dispositif consiste en un plateau sur lequel sont disposés deux supports, dont l'un au moins est mobile, munis de palpeurs réglables en position par rapport au support et d'un élément de maintien de l'attelle à former, une tête d'articulation par exemple.

La position de chacun des palpeurs des deux supports est réglée de façon à reproduire le contour de l'attelle à réaliser. L'attelle, chauffée à la température appropriée, est placée dans le dispositif de formage, et les deux supports sont ensuite mis en contact avec l'attelle de façon à la maintenir à la forme désirée jusqu'à son complet refroidissement.

Les dessins schématiques annexés, qui n'ont pas de caractère limitatif, illustrent ces différentes formes de mise en oeuvre de l'invention. Sur ces dessins :

La figure 1 représente une orthèse d'un type usuel ;

La figure 2 est une vue schématique d'un élément tubulaire conforme à l'invention ;

Les figures 3 à 6 illustrent divers modes d'assemblage d'une attelle conforme à l'invention et d'une articulation ;

La figure 7 illustre un mode d'assemblage de deux attelles et d'une embrasse conformes à l'invention ;

La figure 8 illustre la fabrication d'une attelle conforme à l'invention, possédant directement le profil du membre à appareiller, sans qu'il soit nécessaire de procéder ultérieurement à une mise en forme à chaud de cette attelle ;

Les figures 9, 10, 11, 12, 13 et 14 sont des vues schématiques en coupe de diverses attelles conformes à l'invention ;

La figure 15 est une vue en perspective d'un type d'embrasse conforme à l'invention, illustrant la mise en place de cette embrasse sur l'attelle de la figure 9 ;

La figure 16 représente une orthèse constituée d'éléments modulaires conformes à l'invention ;

La figure 17 représente un dispositif de formage des attelles conforme à l'invention.

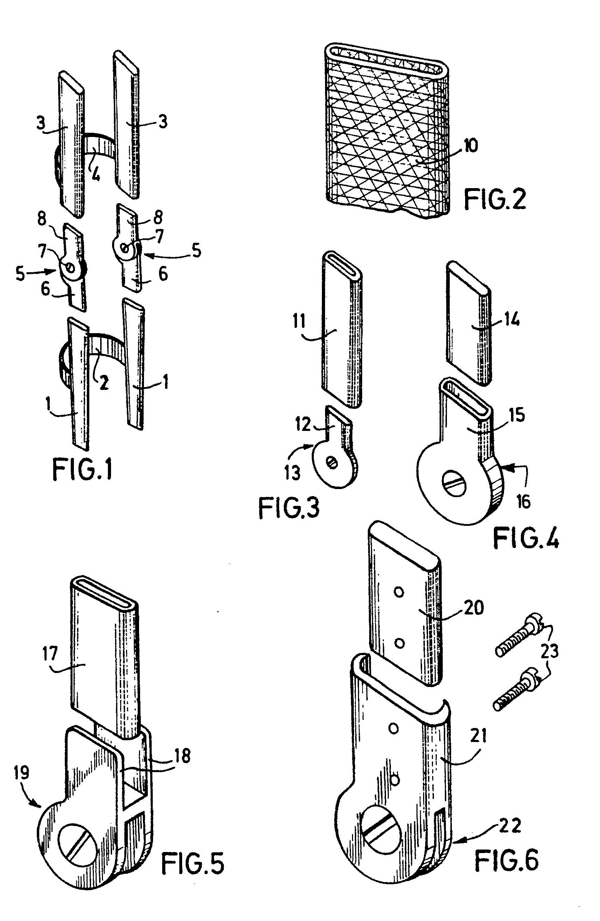

L'orthèse représentée sur la figure 1 est une orthèse pour membres inférieurs d'un type usuel, comprenant deux montants inférieurs 1, réunis par une embrasse 2, et deux montants supérieurs 3, réunis par une embrasse 4.

Les montants supérieurs et inférieurs sont articulés entre eux par un système d'articulation 5, comprenant chacun un raccord 6, apte à être rendu solidaire du montant inférieur 1 et articulé par un axe 7 sur un raccord 8, apte à être rendu solidaire du montant supérieur 3.

Ainsi qu'il a été exposé ci-dessus, aussi bien les montants 1 et 3 que les embrasses 2 et 4 peuvent être constitués, conformément à l'invention, en un matériau composite comprenant des fibres, de préférence tissées, noyées dans une résine thermoplastique polymérisée, ce qui facilite la conformation, la mise à longueur et l'assemblage de ces éléments.

La figure 2 représente une attelle 10 de ce type, comprenant des tresses de fibres tissées inclinées par rapport à l'axe de l'attelle.

Les éléments d'orthèse conformes à l'invention peuvent être réalisés sous forme partiellement ou totalement tubulaire, comme représenté sur la figure 2, ou sous forme d'éléments pleins. Ces éléments peuvent être réalisés à partir d'éléments standards, que l'on met à la forme du membre à équiper par déformation à chaud. Dans le cas où il sont sous forme tubulaire, il est préférable qu'ils comportent un jonc interne de renfort, par exemple en polytétrafluoréthylène, qui peut être retiré de l'élément après la mise en forme définitive.

Les articulations peuvent être réalisées indifféremment en métal, ou en tout matériau approprié, par exemple un matériau composite. Dans ce cas, les montants inférieurs 1 et les raccords 6 peuvent être réalisés en une seule pièce, de même que les montants supérieurs 3 et les raccords 8.

La figure 3 montre un montant tubulaire 11, dans lequel est emmanché un raccord 12 de l'articulation 13. Le raccord 12 et le montant 11 peuvent en outre être assemblés par collage, par exemple à l'aide d'une résine époxyde, par rivetage, vissage, boulonnage ou tout autre moyen connu de la technique.

Comme représenté sur la figure 4, le montant 14, tubulaire ou non, peut également être engagé dans un raccord tubulaire 15 de l'articulation 16.

Sur la figure 5, le montant 17, tubulaire ou non, est logé dans une chape 18 de l'articulation 19.

Enfin, dans la forme de réalisation de la figure 6, le montant 20 est logé dans un élément 21 à section en U de l'articulation 22, et il y est maintenu par des vis 23 ou par tout autre moyen de fixation (colle, rivets etc...).

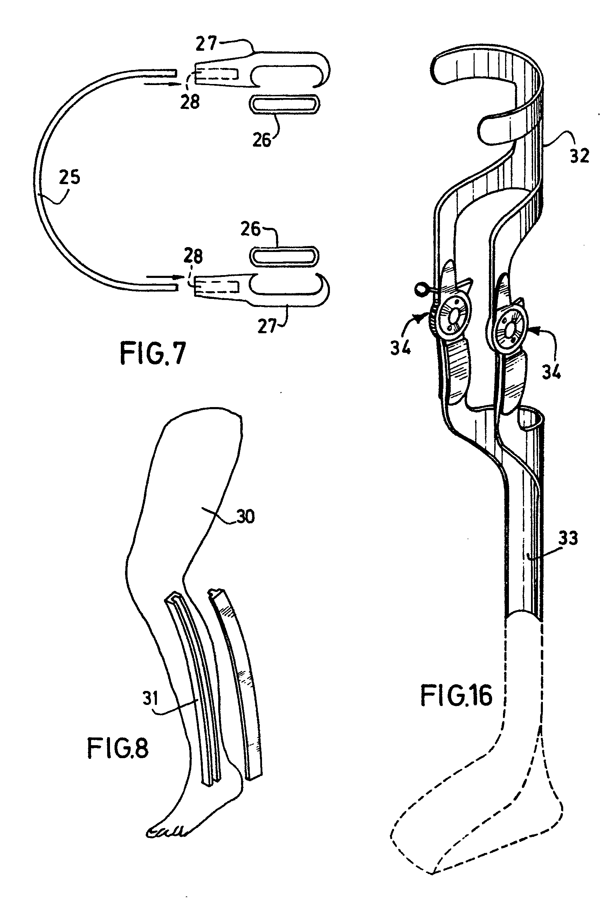

La figure 7 illustre l'assemblage d'une embrasse 25 conforme à l'invention et de deux montants 26. Des organes auxiliaires 27 formant pinces viennent s'encliqueter sur les montants 26, tandis que les extrémités de l'embrasse 25 se logent dans des évidements d'embouts 28 des éléments 27. Les éléments 27 peuvent bien entendu être fixés sur les montants 26 et sur l'embrasse 25 par tout moyen connu dans la technique.

Ainsi qu'il a été exposé ci-dessus, les éléments d'orthèse conformes à l'invention peuvent être réalisés non seulement à partir d'éléments standards, que l'on déforme à chaud pour les mettre à la forme du membre à appareiller, mais être fabriqués également directement sous la forme désirée. Dans ce cas, comme représenté sur la figure 8, on réalise un moulage 30 du membre à appareiller et l'on applique contre le moulage un moule déformable 31 du montant à réaliser. On fait épouser au moule 31 le profil du moulage 30, on y dispose le matériau composite destiné à former le montant et l'on provoque la polymérisation de la résine entrant dans la constitution de ce matériau.

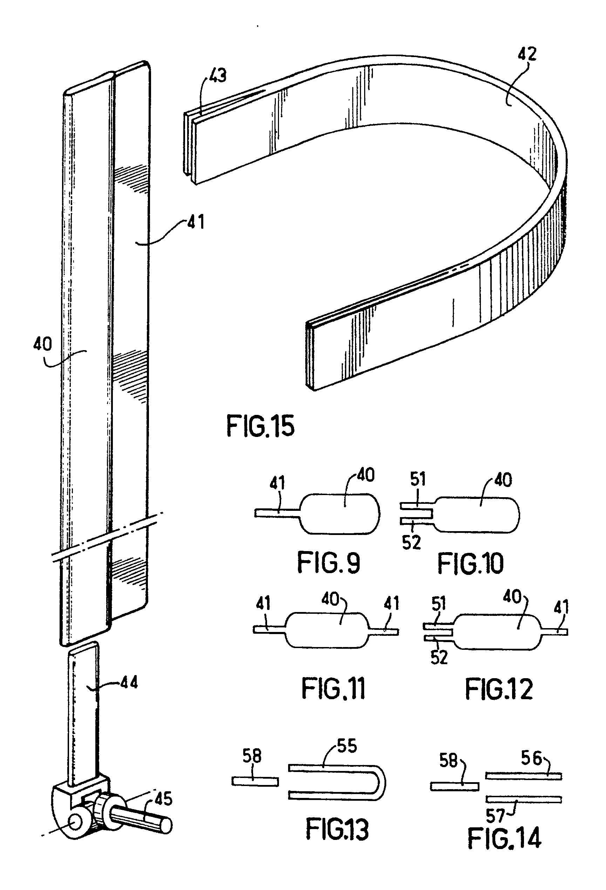

Les figures 9, 10, 11, 12, 13, 14 et 15 illustrent d'autres formes de réalisation de montants pleins ou évidés sur tout ou partie de leur longueur et d'embrasses conformes à l'invention, destinés à faciliter leur mise à dimension et leur assemblage.

Sur les figures 9, 11 et 12, le montant 40 comporte au moins une aile 41 formant nervure, sur laquelle peut être assemblée une embrasse,après mise à longueur, les parties inutilisées de l'aile 41 pouvant ensuite être éliminées par découpage. Comme on le voit sur la figure 15, on peut associer à un tel montant une embrasse 42 fendue dans son épaisseur en 43 à ses deux extrémités (ou constituée de deux parties accolées, solidaires l'une de l'autre uniquement par leur partie médiane). Pour mettre à longueur l'embrasse 42, il suffira d'engager d'une longueur suffisante l'aile 41 dans la fente 43, au besoin en faisant dépasser l'embrasse au-delà de l'aile 41 sur l'attelle 40 proprement dite. Après avoir découpé l'embrasse 42, on y engagera l'aile 41 de la longueur désirée et on assemblera l'embrasse sur cette aile. On pourra ensuite découper les parties inutilisées du montant 40 et de l'aile 41. Dans le cas d'un montant creux 40 on pourra engager dans celui-ci un raccord 44 d'une articulation 45.

Les figures 10 et 12 illustrent un autre montant 40 conforme à l'invention, comportant au moins deux ailes parallèles 51 et 52, entre lesquelles peut être engagée une extrémité d'une embrasse pour en être rendue solidaire par tout moyen d'assemblage connu de la technique. Comme précédemment, on peut découper des parties inutilisées des ailes 51 et 52, après assemblage de l'embrasse. Si celle-ci est trop longue, elle peut également être découpée avant d'être engagée entre les ailes 51 et 52.

Les montants des figures 10 à 12 peuvent être pleins ou évidés sur tout ou partie de leur longueur.

Le montant 55 représenté sur la figure 13 a une section transversale en forme de U. Une embrasse peut être ainsi introduite entre les branches du U et être rendue solidaire de celles-ci par tout moyen d'assemblage connu de la technique.

Le montant de la figure 14 comprend au moins deux lamelles sensiblement parallèles, réunies en au moins deux points de leur longueur et entre lesquelles peut être introduite l'extrémité 58 d'une embrasse pour y être mise à la longueur désirée avant d'être assemblée avec les lamelles 56 et 57, par tout moyen connu de la technique.

Sous toutes ces formes de réalisation, les éléments d'orthèse conformes à l'invention se prêtent donc à une mise à dimension facile par les orthésistes, et à un assemblage tout aussi facile par collage-rivetage-boulonnage ou tout autre moyen connu de la technique, ce qui permet de livrer aux utilisateurs une gamme d'éléments standards, qu'il est facile d'ajuster à la forme et aux dimensions du membre à appareiller.

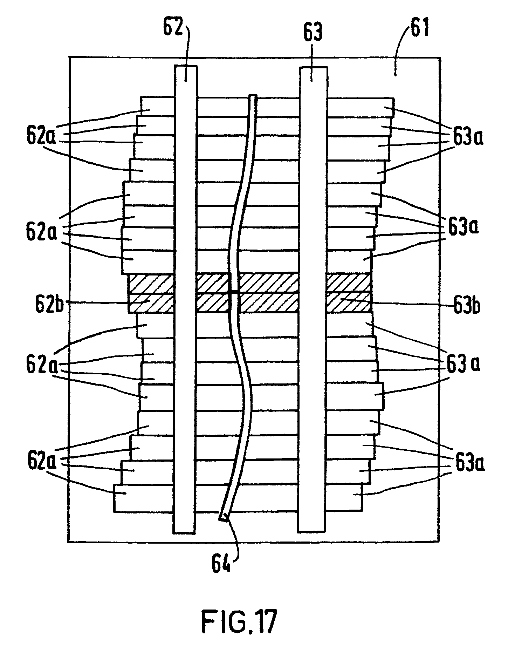

Conformément à l'invention, comme représenté sur la figure 16, il est possible de réaliser des éléments modulaires d'orthèse, 32, 33 en matériau composite, selon des formes et des dimensions standards et qui sont sous forme monobloc au lieu d'être constitués d'organes distincts (attelles, embrasses,...) assemblés entre eux. Ces modules peuvent être adaptés à la conformation exacte du membre du patient et à ses dimensions par thermoformage et par simple découpe. Des organes d'articulation 34 peuvent être rendus solidaires des éléments 32 et 33 par tout moyen connu dans la technique.

Enfin, la figure 17 représente un dispositif de formage des attelles. Celui-ci consiste en un plateau 61 sur lequel sont disposés des supports 62 et 63 dont l'un au moins est mobile sur le plateaux 61 . Ces supports sont munis de palpeurs 62a et 63a, qui sont réglables en position par rapport aux supports,et d'un élément de maintien 62b et 63b de l'attelle 64. Il peut être pratique de maintenir l'attelle par son élément articulaire par exemple. La position de chacun des palpeurs 62a et 63a des supports 62 et 63 est réglée de façon à reproduire le contour de l'attelle 64 à mettre en forme. L'attelle 64 est ensuite chauffée à la température appropriée,puis disposée dans le dispositif de formage préalablement réglé. Les deux supports 62 et 63 sont alors déplacés de façon à mettre en contact avec l'attelle les palpeurs 62a et 63a , pour la mettre en forme,et à la maintenir à cette forme jusqu'à son refroidissement.

Sous ses diverses formes de réalisation, l'invention apporte donc un moyen simple et facile à mettre en oeuvre pour fabriquer rapidement et moyennant un coût réduit des orthèses plus légères que celles de la technique antérieure.

## Revendications

1.  Attelles et embrasses pour la constitution d'orthèses, caractérisées en ce qu'elles sont réalisées en une résine thermoplastique, choisie dans le groupe constitué par les polyesters et les résines acryliques, dans laquelle sont noyées des fibres tissées de carbone, de bore ou d'aramide.

2.  Attelles et embrasses selon la revendication 1, caractérisées en ce que lesdites fibres sont tissées sous forme de tresses.

3.  Attelles et embrasses selon l'une des revendications 1 et 2, caractérisées en ce qu'elles présentent au moins une partie apte à s'emboîter ou à s'encliqueter dans une partie de forme complémentaire d'un autre élément de la même orthèse.

4.  Attelles et embrasses selon l'une des revendications 1 à 3, caractérisées en ce qu'elles comprennent au moins une partie tubulaire.

## Claims

1.  Splints and hoops for making up ortheses characterised in that they are made from a thermoplastic resin selected from the group formed by polyesters and acrylic resins in which are embedded woven fibres of carbon, boron or aramide.

2.  Splints and hoops according to claim 1 characterised in that said fibres are woven in the form of braids.

3.  Splints and hoops according to one of claims 1 and 2 characterised in that they have at least one portion capable of fitting or latching into a portion of complementary shape of another element of the same orthesis.

4.  Splints and hoops according to one of claims 1 to 3 characterised in that they comprise at least one tubular portion.

## Patentansprüche

1.  Schienen und Stützen zur Bildung von Orthesen, dadurch gekennzeichnet, daß sie aus Thermoplasten, ausgewählt aus der Gruppe der Polyester und Acrylharze, in welchen gewbte Fasern aus Kohlenstoff, Bor oder Aramid eingebettet sind, ausgebildet sind.

2.  Schienen und Stützen nach Anspruch 1, dadurch gekennzeichnet, daß die Fasern in Form von Tressen gewebt sind.

3.  Schienen und Stützen nach Anspruch 1 oder 2, dadurch gekennzeichnet, daß sie wenigstens einen Bereich aufweisen, welcher in einem Bereich komplementärer Form einem anderen Elementes derselben Orthese einfügbar oder verriegelbar ist.

4.  Schienen und Stützen nach einem der Ansprüche 1 bis 3, dadurch gekennzeichnet, daß sie wenigstens einen rohrförmigen Bereich umfassen.

FIG.1

FIG.2

FIG.3

FIG.4

FIG.5

FIG.6

FIG.7

FIG.8

FIG.16

FIG.15

40

41

43

42

44

45

41 40

FIG.9

51 40

52

FIG.10

41 40 41

FIG.11

51 40 41

52

FIG.12

58 55

FIG.13

58 56

57

FIG.14

FIG.17